# EUROPÄISCHE PATENTSCHRIFT

(11) **EP 0 421 216 B1**
(45) Veröffentlichungstag und Bekanntmachung des Hinweises auf die Patenterteilung: **27.10.1993**
(21) Anmeldenummer: 90118259.2
(22) Anmeldetag: 22.09.1990
(51) Int. Cl.: C07C 209/74

(54) **Verfahren zur Herstellung von 2,4- bzw. 2,6-Dihalogen-anilin**
Process for the preparation of 2,4, resp. 2,6 dihalo-aniline
Procédé pour la préparation de 2,4-resp. 2,6 dihalogéno-aniline

(30) Priorität: 04.10.1989 DE 3933093
(43) Veröffentlichungstag der Anmeldung: 10.04.1991
(73) Patentinhaber: BAYER AG, 51368 Leverkusen (DE)
(72) Erfinder: Kissener, Wolfram, Dr., D-5060 Bergisch-Gladbach 2 (DE); Fiege, Helmut, Dr., D-5090 Leverkusen (DE)

(56) Entgegenhaltungen:
- EP-A- 0 055 629
- ORGANIC SYNTHESES, Band 24, Seiten 47-53, John Wiley & Sons, Inc., New York, US; M.K. SEIKEL: "2,6-Dichloroaniline and 2,6-dibromoaniline"
- RECEUIL DES TRAVAUX CHIMIQUES DES PAYS-BAS, Band 78, Nr. 7, Juli 1959, Seiten 534-542; C. VAN DER STELT et al.: "The chlorination of 4-aminobenzoic acid and its methyl ester"
- CHEMICAL ABSTRACTS, Band 97, Nr. 19, 8. November 1982, Seite 675, Zusammenfassung Nr. 162558t, Columbus, Ohio, US; & ES-A-503 386

## Beschreibung

Die vorliegende Erfindung betrifft ein Verfahren zur Herstellung von 2,4- bzw. 2,6-Dihalogen-anilin oder -anilinderivaten durch Umsetzung eines Amino-benzoesäureesters mit einem Chlorierungs- oder Bromierungsmittel und anschließender Verseifung und Decarboxylierung.

2,4- bzw. 2,6-Dichlor- und Dibrom-anilin sind wichtige Zwischenprodukte zur Herstellung von Arzneimitteln, Farbstoffen und Pflanzenschutzmitteln.

Es ist bekannt, 2,6-Dichloranilin in einem zweistufigen Verfahren durch Umsetzung von Sulfanilamid mit der äquivalenten Menge 30 %igem Wasserstoffperoxid in halbkonzentrierter Salzsäure bei 60°C und anschließender Verseifung des so erhaltenen 3,5-Dichlorsulfanilamids in 70 %iger Schwefelsäure bei 165-195°C herzustellen (Org. Synt. Coll. Vol. III (1955), 262). Das auf diese Art hergestellte Produkt ist jedoch nicht rein und erfordert vor dem Einsatz für weitere Syntheseschritte eine Reinigung. Gemäß DD 64 061 wird dieses Verfahren durch Zwischenisolierung des chlorierten Sulfanilamids überarbeitet; die Ausbeute ist jedoch mit 62,5 % über beide Verfahrensstufen unbefriedigend niedrig. In DD 247 670 ist ein weiteres Verfahren zur Herstellung von 2,6-Dichloranilin, ebenfalls ausgehend von Sulfanilamid, beschrieben. In diesem Verfahren wird gleichzeitig 2,4,6-Trichloranilin erhalten und isoliert; die Ausbeute an reinem 2,6-Dichloranilin ist jedoch ebenfalls niedrig.

Eine weitere Herstellungsmethode für 2,6-Dichloranilin ist in ES 503 386, zitiert nach C. A. 97 (1982), 162.558 t, beschrieben. Hierbei wird 3,5-Dichlor-4-amino-benzoesäure mit Kaliumbromat zu 3,5-Dichlor-4-bromanilin umgesetzt und dann mit Zink in alkalischer Lösung reduziert. Die Herstellung von reiner 3,5-Dichlor-4-amino-benzoesäure als Ausgangsprodukt ist jedoch nur in schlechten Ausbeuten möglich (Rec. 78 (1959) 534 und Chem. Ber. 74 (1941), 807).

In der JP-Patentanmeldung 57/169.447 (1982) ist die Darstellung von 2,6-Dichloranilin, ausgehend von 3,5-Dichlor-brombenzol beschrieben, welches nitriert und mit Wasserstoff katalytisch zu Anilin hydriert wird. Da die Nitrogruppe in p- und o-Stellung zum Brom eintreten kann, erhält man ein Gemisch von 2,6- und 2,4-Dichloranilin.

Es wurde ein Verfahren zur Herstellung von 2,4- bzw. 2,6-Dihalogen-anilin oder -anilinderivaten gefunden, das dadurch gekennzeichnet ist, daß man einen Amino-benzoesäureester der Formel 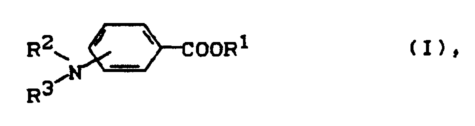
in der
- R¹: geradkettiges oder verzweigten C₁-C₈-Alkyl, Benzyl oder Phenyl bedeutet und
- R² und R³: unabhängig voneinander für Wasserstoff, geradkettiges oder verzweigtes C₁-C₈-Alkyl oder Benzyl stehen, wobei R² und R³ gemeinsam -(-CH₂-)₄-, -(-CH₂-)₅-, -C₂H₄-O-C₂H₄-, -C₂H₄-NH-C₂H₄- oder -C₂H₄-NCH₃-C₂H₄- bedeuten können,

mit 2-2,5 Mol eines Chlorierungs- oder Bromierungsmittels in einem inerten Reaktionsmedium bei einer Temperatur von 40-160°C umsetzt und anschließend den dihalogenierten Amino-benzoesäureester verseift und decarboxyliert.

Geradkettiges oder verzweigtes C₁-C₈-Alkyl ist beispielsweise Methyl, Ethyl, Propyl, Isopropyl, Butyl, Isobutyl, tert.-Butyl, die isomeren Amyle, Hexyle oder Octyle. Hiervon sind die genannten C₁-C₄-Alkylreste bevorzugt; besonders bevorzugt sind Ethyl und Isobutyl.

Benzyl im aromatischen Teil oder Phenyl können durch C₁-C₄-Alkyl der genannten Art, bevorzugt durch Methyl, sowie durch Fluor, Chlor oder Brom oder durch C₁-C₄-Alkoxy, bevorzugt durch Methoxy, substituiert sein.

Das N-Atom der Aminogruppe kann in der genannten Weise substituiert sein.

In bevorzugter Weise werden 4-Amino-benzoesäureester der Formel 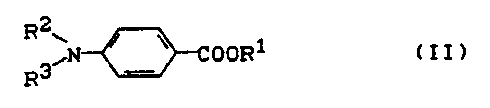
eingesetzt, in der
R¹, R² und R³ die oben genannte Bedeutung haben.

In weiterhin bevorzugter Weise werden Amino-benzoesäureester der Formeln (I) und (II) eingesetzt, in denen an die Stelle von R¹ der Rest R¹¹ mit der Bedeutung eines geradkettigen oder verzweigten C₁-C₄-Alkyls oder Phenyl tritt. In besonders bevorzugter Weise werden Amino-benzoesäureester der Formeln (I) und (II) eingesetzt, in denen an die Stelle von R¹¹ der Rest R²¹ mit der Bedeutung eines geradkettigen oder verzweigten C₁-C₄-Alkylrestes tritt.

In weiterhin bevorzugter Weise werden Amino-benzoesäureester der Formeln (I) und (II) eingesetzt, in denen an die Stelle von R² und R³ die Reste R¹² und R¹³ treten, von denen R¹² Wasserstoff, geradkettiges oder verzweigtes C₁-C₄-Alkyl oder Benzyl bedeutet und R¹³ für Wasserstoff, Methyl oder Ethyl steht. In besonders bevorzugter Weise werden Amino-benzoesäureester der Formeln (I) und (II) eingesetzt, in denen an die Stelle von R¹² der Rest R²² mit der Bedeutung von Wasserstoff, Methyl oder Ethyl tritt und in denen an die Stelle von R¹³ Wasserstoff tritt.

Beispiele für erfindungsgemäß einsetzbare Amino-benzoesäureester sind 4-Amino-benzoesäure-methylester, 4-Amino-benzoesäure-ethylester, 4-Amino-benzoesäure-isopropylester, 4-Amino-benzoesäure-isobutylester, 4-Amino-benzoesäure-phenylester.

Die Darstellung von Amino-benzoesäureestern der genannten Art kann in einer dem Fachmann bekannten Weise, beispielsweise durch Veresterung von Nitrobenzoesäure mit einem Alkohol oder mit Phenol und anschließender Hydrierung oder durch Veresterung von 4-Aminobenzoesäure erfolgen. Die Aminogruppe kann weiterhin in bekannter Weise mit den obigen Resten R² und R³ alkyliert werden (vgl. Houben-Weyl, Methoden der Organischen Chemie VIII (1952), 516 bzw. XI/1 (1957), 360).

Bei Amino-benzoesäureestern mit p-ständiger Aminogruppe erhält man demnach 2,6-Dihalogen-anilin. Bei o-ständiger Aminogruppe erhält man 2,4-Dihalogen-anilin. Bei m-ständiger Aminogruppe erhält man im wesentlichen 2,6-Dihalogen-anilin neben etwas 2,4-Dihalogen-anilin. Am wichtigsten ist das erfindungsgemäße Verfahren für die Umsetzung von Amino-benzoesäureestern mit p-ständiger Aminogruppe.

Die erfindungsgemäß erhältlichen 2,4- bzw. 2,6-Dihalogen-aniline sind demnach solche der Formeln 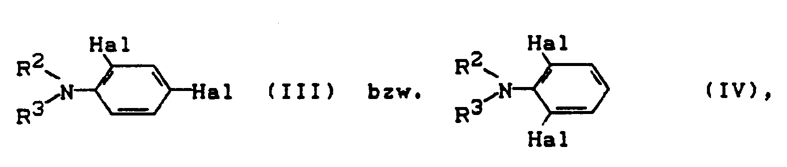
in denen
R² und R³ die obige Bedeutung haben und
Hal für Chlor oder Brom steht.

Als Chlorierungs- oder Bromierungsmittel seien beispielsweise Chlor, Sulfurylchlorid, Chlorwasserstoff/Was serstoffperoxid, Brom, Sulfurylbromid oder Bromwasserstoff/Wasserstoffperoxid, in bevorzugter Weise Chlor oder Brom genannt.

Solche Chlorierungs- oder Bromierungsmittel werden in einer Menge von 2-2,5 Mol, bevorzugt 2-2,1 Mol, besonders bevorzugt 2-2,05 Mol, pro Mol des Amino-benzoesäureesters eingesetzt. In weiterhin bevorzugter Weise ist die Halogenierung eine Chlorierung.

Als inertes Reaktionsmedium kommen aliphatische oder aromatische Halogenkohlenwasserstoffe in Frage, wie Chlorbenzol, Dichlorbenzol, Tetrachlorkohlenwasserstoff, Brombenzol oder ähnliche, die dem Fachmann für solche Zwecke bekannt sind. Das inerte Reaktionsmedium kann auch aus mehreren Lösungs/-Verdünnungsmitteln der genannten Art bestehen. Das inerte Reaktionsmedium wird in einer Menge von 50-3.000, bevorzugt 100-1.500, besonders bevorzugt 300-1.000 ml, pro Mol des Amino-benzoesäureesters eingesetzt.

Die erfindungsgemäße Chlorierung bzw. Bromierung wird bei einer Temperatur von 40-160°C, bevorzugt 60-140°C, besonders bevorzugt 70-110°C, durchgeführt.

Die Chlorierungs- oder Bromierungsreaktion kann beispielsweise so durchgeführt werden, das der Aminobenzoesäureester im inerten Reaktionsmedium vorgelegt wird, worauf der Reaktionsansatz unter Rühren auf die gewählte Reaktionstemperatur gebracht wird. Sollte die gewählte Reaktionstemperatur oberhalb des Siedepunktes des Reaktionsmediums liegen, kann in einer dem Fachmann bekannten Art bei erhöhtem Druck gearbeitet werden. Ansonsten ist das erfindungsgemäße Verfahren vom Druck unabhängig. Bei der gewählten Reaktionstemperatur wird dann das Chlorierungsmittel, am günstigsten im Maße seines Verbrauchs, eingeleitet. Nach dem Ausblasen von Resten des Halogenierungsmittels und seiner Umsetzungsprodukte kann das Reaktionsprodukt durch bloßes Abdestillieren des Reaktionsmediums isoliert werden. Es ist jedoch auch möglich, das inerte Reaktionsmedium vorzulegen und den Amino-benzoesäureester und das Halogenierungsmittel simultan einzuleiten; bei dieser Variante kann auch ein Teil des Amino-benzoesäureesters vorgelegt werden, während der Rest simultan zum Halogenierungsmittel eingeleitet wird. Ein solches simultanes Einleiten kann auch bereits vor Erreichen der gewählten Reaktionstemperatur begonnen werden; durch Erwärmen des Reaktionsansatzes wird sodann noch während des simultanen Einleitens die Reaktionstemperatur erreicht.

Bei der Halogenierung kann grundsätzlich ein Katalysator wie FeCl₃, TiCl₄ oder ZnCl₂ eingesetzt werden. Solche Katalysatoren bringen jedoch keinen weiteren Vorteil, und es ist sogar ein besonderes Merkmal der erfindungsgemäßen Halogenierung, daß sie ohne solche Katalysatoren durchgeführt werden kann.

Es ist ein weiteres besonderes Merkmal der erfindungsgemäßen Halogenierung, das sie auch an Substraten der Formel (I) bzw. (II) durchgeführt werden kann, in denen einer oder beide Reste R² und R³ die Bedeutung Wasserstoff annehmen, ohne daß eine unerwünschte N-Halogenierung eintritt, was für gewöhnlich durch Schutzgruppen, wie eine Acetylgruppe, verhindert werden muß, die anschließend in einem separaten Reaktionsschritt erst wieder entfernt werden muß.

Der zweifach chlorierte bzw. bromierte Amino-benzoesäureester wird sodann verseift und decarboxyliert.

Hierbei kann so vorgegangen werden, daß der zweifach chlorierte bzw. bromierte Amino-benzoesäureester alkalisch oder sauer in einer dem Fachmann grundsätzlich bekannten Art zur zugrundeliegenden Carbonsäure verseift wird. Diese Carbonsäure wird anschließend in einem bezüglich der Decarboxylierung inerten Lösungs-/Verdünnungsmittel bei 100-400°C, bevorzugt 150-300°C, besonders bevorzugt 180-280°C, decarboxyliert.

Lösungs-/Verdünnungsmittel können polare organische Verbindungen, organische oder anorganische Säuren sowie Wasser sein. Beispiele hierfür sind: Dimethylformamid, Sulfolan, Dimethylsulfoxid, Essigsäure, Salzsäure, Schwefelsaure; in bevorzugter Weise wird in Wasser gearbeitet. Solche Lösungs-/Verdünnungsmittel oder ein Gemisch mehrerer von ihnen werden in einer Menge von 50-2.000, bevorzugt 200-1.500, besonders bevorzugt 500-1.300 ml, pro Mol der 2fach chlorierten bzw. bromierten Amino-benzoesäure eingesetzt.

Diese Decarboxylierung ist grundsäztlich vom Druck unabhängig. Ein erhöhter Druck wird daher nur angewandt, wenn die gewählte Arbeitstemperatur oberhalb des Siedepunktes des Lösungs-/Verdünnungsmittels liegt. In einem solchen Fall kann beispielsweise in einem Rührautoklaven unter dem sich automatisch einstellbaren Druck gearbeitet werden.

Für den Fall, daß die zweifach chlorierte bzw. bromierte Amino-benzoesäure bei der gewählten Arbeitstemperatur flüssig ist, kann grundsätzlich auch auf ein Lösungs/Verdünnungsmittel verzichtet werden. Im Sinne einer gleichmäßigen Reaktionsführung ist es jedoch bevorzugt, ein Lösungs-/Verdünnungsmittel der obigen Art und in der obigen Menge zu verwenden.

Grundsätzlich ist es möglich, die Decarboxylierung in Gegenwart sauer reagierender Verbindungen, beispielsweise Brönstedt- oder Lewissäuren, Bleicherden oder sauer reagierenden Ionenaustauschern oder Zeolithen durchzuführen, sofern das gewählte Lösungs-/Verdünnungsmittel nicht selbst eine Säure ist. Da es jedoch grundsätzlich möglich ist, auch in Gegenwart eines nichtsauren Lösungs-/Verdünnungsmittels zu arbeiten, ist ein solcher Zusatz entbehrlich und wird wegen der vereinfachten Aufarbeitung bevorzugt fortgelassen.

Nach der Decarboxylierung kann das 2,4- bzw. 2,6-Dichlor(brom)anilin durch Kristallisation, Lösungsmittelextraktion, Destillation oder Wasserdampfdestillation aus dem Decarboxylierungsansatz gewonnen werden.

Der glatte Verlauf der Decarboxylierung in hohen Raum-Zeit-Ausbeuten und bei Erzielung hoher Reinheiten ist überraschend, da bekannt ist, daß die Decarboxylierung nichtaktivierter aromatischer Carbonsäuren sehr hohe Reaktionstemperaturen, lange Reaktionszeiten bzw. Zusätze an Katalysatoren, wie Metallsalzen, erfordert (Houben-Weyl, Bd. E5 (1985), 468 sowie Bd. VIII (1952), 494; EP 55 629).

In vorteilhafter Weise kann jedoch die Verseifung und Decarboxylierung in einem Schritt vorgenommen werden. Hierzu wird in Wasser oder einem Wasser enthaltenden Lösungs-/Verdünnungsmittel bei 100-400°C, bevorzugt 150-300°C, besonders bevorzugt 180-280°C, gearbeitet.

Als mit dem Wasser verwendbare Lösungs-/Verdünnungsmittel kommen aliphatische oder aromatische Kohlenwasserstoffe, aliphatische oder aromatische Halogenkohlenwasserstoffe oder organische oder anorganische Säuren in Frage. Solche mit Wasser gemeinsam einzusetzende Lösungs-/Verdünnungsmittel sind beispielsweise: Toluol, Chlorbenzol, Essigsäure, Salzsäure, Schwefelsäure. In bevorzugter Weise wird jedoch in Wasser allein gearbeitet, wobei sich der Vorteil einer vereinfachten Aufarbeitung des Reaktionsgemisches einstellt. Ein Zusatz von ganz geringen Mengen von Säure, um einen pH-Wert von 4-6,5 einzustellen kann wünschenswert sein; es ist jedoch grundsäztlich möglich, auf alle solche Zusätze zu verzichten. Wasser oder ein Wasser enthaltendes Lösungs-/Verdünnungsmittel wird in einer Menge von 50-2.000, bevorzugt 200-1.500, besonders bevorzugt 500-1.300 ml, pro Mol des zweifach chlorierten bzw. bromierten Aminobenzoesäureesters eingesetzt.

Bei der bevorzugten gleichzeitigen Verseifung und Decarboxylierung in Wasser ist Druck erforderlich, um die Reaktionstemperatur zu erreichen und ein Abdestillieren des Wassers zu verhindern. Dieser Druck ist in bevorzugter Weise der Eigendruck des Systems, der sich in einer dem Fachmann bekannten Weise von selbst einstellt. Grundsätzlich ist es möglich, einen zusätzlichen Druck durch Inertgas, wie Stickstoff, Wasserstoff, Edelgase oder ähnliche anzubringen; in der Regel wird man aus Vereinfachungsgründen darauf verzichten. Durch die Spaltprodukte der Verseifung und Decarboxylierung steigt der anfängliche Druck. Dieser erhöhte Druck kann durch teilweises Entspannen abgebaut werden.

Die Aufarbeitung eines solchen Verseifungs- und Decarboxylierungsgemisches geschieht in der oben geschilderten Weise.

### Beispiel 1

1 Mol = 151 g 4-Amino-benzoesäure-methylester wurden in 1 l Chlorbenzol vorgelegt. Man erwärmte auf 100°C und leitete bei dieser Temperatur über vier Stunden 2 Mol = 142 g Chlor ein. Anschließend destillierte man das Lösungsmittel im Vakuum ab. Es verblieben 221 g 3,5-Dichlor-4-amino-benzoesäure-methylester, entsprechend einer Ausbeute von 92,4 %.

0,2 Mol = 48 g 3,5-Dichlor-4-amino-benzoesäure-methylester wurden zusammen mit 250 ml Wasser in einem Autoklaven vorgelegt. Man erwärmte auf 250°C und rührte drei Stunden bei dieser Temperatur. Entstehendes Reaktionsgas wurde kontinuierlich bis zu einem Druck von ca. 40 bar abgelassen. Das Produkt wurde durch Wasserdampfdestillation gewonnen. Man erhielt 26,9 g 2,6-Dichloranilin, entsprechend einer Ausbeute von 75,3 %.

### Beispiel 2

1 Mol = 165 g 4-Amino-benzoesäure-ethylester wurden in 1 l Chlorbenzol vorgelegt. Man erwärmte auf 100°C und leitete über drei Stunden 2 Mol = 142 g Chlor ein. Das Lösungsmittel wurde im Vakuum vollständig abdestilliert und es verblieben 232 g 3,5-Dichlor-4-amino-benzoesäureethylester, entsprechend einer Ausbeute von 94 %.

0,2 Mol = 49,3 g 3,5-Dichlor-4-amino-benzoesäure-ethylester wurden zusammen mit 250 ml Wasser in einem Autoklaven vorgelegt. Man erwärmte auf 250°C und rührte 2,5 Stunden bei dieser Temperatur. Entstehendes Reaktionsgas wurde kontinuierlich bis zu einem Druck von ca. 40 bar abgenommen. Das Produkt wurde anschließend durch Wasserdampfdestillation gewonnen. Man erhielt 31 g 2,6-Dichloranilin, entsprechend einer Ausbeute von 94 %.

### Beispiel 3

1 Mol = 194 g 4-Amino-benzoesäure-isobutylester wurden in 1 l Chlorbenzol vorgelegt. Man erwärmte auf 90°C und leitete bei dieser Temperatur über vier Stunden 2 Mol = 142 g Chlor ein. Nach dem Abdestillieren des Lösungsmittels im Vakuum verblieben 260 g 3,5-Dichlor-4-aminobenzoesäure-isobutylester, entsprechend einer Ausbeute von 94 %.

0,2 Mol = 55,2 g 3,5-Dichlor-4-amino-benzoesäure-isobutylester wurden zusammen mit 250 ml Wasser in einem Autoklaven vorgelegt. Man rührte 2,5 Stunden bei 250°C und nahm das entstehende Reaktionsgas kontinuierlich bis zu einem Druck von ca. 40 bar ab. Das Produkt wurde durch Wasserdampfdestillation gewonnen. Man erhielt 32 g 2,6-Dichloranilin, entsprechend einer Ausbeute von 98 %.

### Beispiel 4

49 g (0,174 Mol) 3,5-Dichlor-4-amino-benzoesäure-phenylester wurden zusammen mit 200 ml Waaser in einem Autoklaven vorgelegt. Man erwärmte auf 250°C und rührte acht Stunden bei dieser Temperatur. Während der Reaktion wurde das Reaktionsgas kontinuierlich abgelassen und ein Druck von ca. 41 bar eingestellt. Der Rückstand wurde mit Wasserdampf destilliert, und man erhielt 35,8 g 2,6-Dichloranilin mit einem Gehalt von 63 %. Dies entspricht einer Ausbeute von 80 %.

### Beispiel 5

0,2 Mol = 41,2 g 3,5-Dichlor-4-amino-benzoesäure wurden zusammen mit 250 ml Wasser in einem Autoklaven vorgelegt. Man erwärmte auf 250°C und rührte acht Stunden bei dieser Temperatur. Anschließend wurde das Produkt durch Wasserdampfdestillation gewonnen. Man erhielt 26 g 2,6-Dichloranilin in einer Reinheit von 99,8 %, entsprechend einer Ausbeute von 80 %.

## Patentansprüche

1. Verfahren zur Herstellung von 2,4- bzw. 2,6-Dihalogen-anilin oder -anilinderivaten, dadurch gekennzeichnet, das man einen Amino-benzoesäureester der Formel 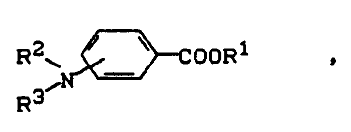 in der
R¹ geradkettiges oder verzweigten C₁-C₈-Alkyl, Benzyl oder Phenyl bedeutet und
R² und R³ unabhängig voneinander für Wasserstoff, geradkettiges oder verzweigtes C₁-C₈-Alkyl oder Benzyl stehen, wobei R² und R³ gemeinsam -(-CH₂-)₄-, -(-CH₂-)₅-, -C₂H₄-O-C₂H₄-, -C₂H₄-NH-C₂H₄- oder -C₂H₄-NCH₃-C₂H₄- bedeuten können,
mit 2-2,5 Mol eines Chlorierungs- oder Bromierungsmittels in einem inerten Reaktionsmedium bei einer Temperatur von 40-160°C umsetzt und anschließend den dihalogenierten Amino-benzoesäureester verseift und decarboxyliert.

2. Verfahren nach Anspruch 1, dadurch gekennzeichnet, das als Amino-benzoesäureester einer der Formel 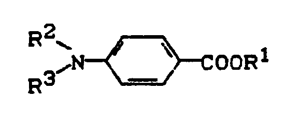 eingesetzt wird, in welchem
R¹, R², und R³ die in Anspruch 1 genannte Bedeutung haben.

3. Verfahren nach Anspruch 1, dadurch gekennzeichnet, daß als Chlorierungs- oder Bromierungsmittel elementares Chlor oder Brom eingesetzt wird.

4. Verfahren nach Anspruch 1, dadurch gekennzeichnet, das die Halogenierung eine Chlorierung ist.

5. Verfahren nach Anspruch 1, dadurch gekennzeichnet, das der zweifach chlorierte bzw. bromierte Aminobenzoesäureester alkalisch oder sauer zur zugrundeliegenden Carbonsäure verseift wird und diese Carbonsäure anschließend in einem bezüglich der Decarboxylierung inerte Lösungs-/Verdünnungsmittel bei 100-400°C, bevorzugt 150-300°C, besonders bevorzugt 180-280°C, decarboxyliert wird.

6. Verfahren nach Anspruch 5, dadurch gekennzeichnet, das in Dimethylformamid, Sulfolan, Dimethylsulfoxid, Essigsäure, Salzsäure, Schwefelsäure, Wasser oder einem Gemisch von ihnen, bevorzugt in Wasser, in einer Menge von 50-2.000, bevorzugt 200-1.500, besonders bevorzugt 500-1.300 ml, pro Mol der Carbonsäure gearbeitet wird.

7. Verfahren nach Anspruch 1, dadurch gekennzeichnet, das die Verseifung und Decarboxylierung in einem Schritt in Wasser oder einem Wasser enthaltenden Lösungs-/Verdünnungsmittel bei 100-400°C, bevorzugt 150-300°C, besonders bevorzugt 180-280°C, durchgeführt wird.

## Claims

1. Process for the preparation of 2,4- or 2,6-dihalogeno-aniline or -aniline derivatives, characterised in that an amino-benzoic acid ester of the formula 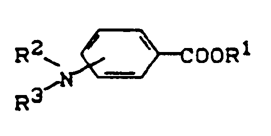 in which
R¹ denotes straight-chain or branched C₁-C₈-alkyl, benzyl or phenyl and
R² and R³, independently of one another, represent hydrogen, straight-chain or branched C₁-C₈-alkyl or benzyl, where R² and R³ together may denote -(CH₂-)₄-, -(CH₂-)₅-, -C₂H₄-O-C₂H₄-, -C₂H₄-NH-C₂H₄-, or -C₂H₄-NCH₃-C₂H₄-,
is reacted with 2-2.5 moles of a chlorinating or brominating agent in an inert reaction medium at a temperature of 40-160°C and the dihalogenated aminobenzoic acid ester is subsequently hydrolyzed and decarboxylated.

2. Process according to Claim 1, characterised in that, as an amino-benzoic acid ester, one of the formula 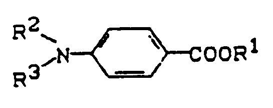 is employed in which
R¹, R² and R³ have the meaning mentioned in Claim 1.

3. Process according to Claim 1, characterised in that elementary chlorine or bromine is employed as the chlorinating or brominating agent.

4. Process according to Claim 1, characterised in that the halogenation is a chlorination.

5. Process according to Claim 1, characterised in that the doubly chlorinated or brominated amino-benzoic acid ester is hydrolyzed to give the carboxylic acid on which it is based under alkaline or acid conditions and this carboxylic acid is subsequently decarboxylated at 100-400°C, preferably 150-300°C, particularly preferably 180-280°C, in a solvent/diluent which is inert with respect to the decarboxylation.

6. Process according to Claim 5, characterised in that it is carried out in dimethylformamide, sulpholane, dimethyl sulphoxide, acetic acid, hydrochloric acid, sulphuric acid, water or a mixture thereof, preferably in water, in an amount of 50-2,000, preferably 200-1,500, particularly preferably 500-1,300 ml, per mole of the carboxylic acid.

7. Process according to Claim 1, characterised in that the hydrolysis and decarboxylation is carried out in one step in water or a water-containing solvent/diluent at 100-400°C, preferably 150-300°C, particularly preferably 180-280°C.

## Revendications

1. Procédé de production de 2,4- ou 2,6-dihalogéno-aniline ou de ses dérivés, caractérisé en ce qu'on fait réagir un ester d'acide aminobenzoïque de formule 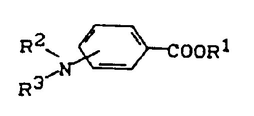 dans laquelle
R¹ est un groupe alkyle en C₁ à C₈ à chaîne droite ou ramifiée, benzyle ou phényle et
R² et R³ représentent, indépendamment l'un de l'autre, l'hydrogène, un groupe alkyle en C₁ à C₈ à chaîne droite ou ramifiée ou un groupe benzyle, R² et R³ pouvant former conjointement un groupe -(-CH₂-)₄-, -(-CH₂-)₅-, -C₂ H₄-O-C₂H₄-, -C₂H₄-NH-C₂H₄- ou -C₂H₄-NCH₃ -C₂H₄-,
avec 2 à 2,5 moles d'un agent de chloration ou de bromation dans un milieu réactionnel inerte, à une température de 40 à 160°C, puis on saponifie l'ester d'acide aminobenzoïque dihalogéné et on le décarboxyle.

2. Procédé suivant la revendication 1, caractérisé en ce qu'on utilise comme ester d'acide aminobenzoïque un ester de formule 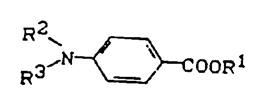 dans laquelle
R¹, R² et R³ ont la définition indiquée dans la revendication 1.

3. Procédé suivant la revendication 1, caractérisé en ce qu'on utilise comme agent de chloration ou de bromation le chlore ou le brome élémentaire.

4. Procédé suivant la revendication 1, caractérisé en ce que l'halogénation est une chloration.

5. Procédé suivant la revendication 1, caractérisé en ce que l'ester d'acide aminobenzoïque dichloré ou dibromé est saponifié par voie alcaline ou acide en l'acide carboxylique de base et cet acide carboxylique est ensuite décarboxylé dans un solvant/diluant inerte vis-à-vis de la décarboxylation, à 100-400°C, de préférence à 150-300°C, notamment à 180-280°C.

6. Procédé suivant la revendication 5, caractérisé en ce qu'on opère dans le diméthylformamide, le sulfolane, le diméthylsulfoxyde, l'acide acétique, l'acide chlorhydrique, l'acide sulfurique, l'eau ou un mélange de ces composés, de préférence dans l'eau, en une quantité de 50 à 2000, de préférence de 200 à 1500, notamment de 500 à 1300 ml par mole de l'acide carboxylique.

7. Procédé suivant la revendication 1, caractérisé en ce qu'on conduit la saponification et la décarboxylation en une seule étape dans l'eau ou dans un solvant/diluant contenant de l'eau, à 100-400°C, de préférence à 150-300°C, notamment à 180-280°C.
